(19) **Europäisches Patentamt / European Patent Office / Office européen des brevets**

(11) **EP 3 563 148 B1**

(12) **EUROPEAN PATENT SPECIFICATION**

(45) Date of publication and mention
of the grant of the patent:
**01.10.2025 Bulletin 2025/40**

(21) Application number: **17822697.3**

(22) Date of filing: **22.12.2017**

(51) International Patent Classification (IPC):
*C12M 1/36* $^{(2006.01)}$     *C12M 1/00* $^{(2006.01)}$
*G01N 30/46* $^{(2006.01)}$     *B01D 15/18* $^{(2006.01)}$

(52) Cooperative Patent Classification (CPC):
**G01N 30/468; B01D 15/1821; C12M 41/48;**
**C12M 47/12**

(86) International application number:
**PCT/EP2017/084495**

(87) International publication number:
**WO 2018/122196 (05.07.2018 Gazette 2018/27)**

(54) **BIOPROCESS PURIFICATION SYSTEM AND METHOD**

REINIGUNGSSYSTEM UND REINIGUNGSVERFAHREN FÜR BIOPROZESS

MÉTHODE ET SYSTÈME DE PURIFICATION POUR BIOTRAITEMENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **29.12.2016 GB 201622343**

(43) Date of publication of application:
**06.11.2019 Bulletin 2019/45**

(73) Proprietor: **Cytiva Sweden AB**
**751 84 Uppsala (SE)**

(72) Inventors:
• **HYCKENBERG, Key**
**751 84 Uppsala (SE)**
• **BERG, Mikael. Johan Helmer Eugen**
**751 84 Uppsala (SE)**
• **SKOGLAR, Helena**
**751 84 Uppsala (SE)**

• **MALMQUIST, Gunnar**
**751 84 Uppsala (SE)**
• **MATHIASSON, Linda**
**751 84 Uppsala (SE)**
• **MATTSSON, Lars, Henning Ivar**
**751 84 Uppsala (SE)**
• **SENDABO, Sara**
**751 84 Uppsala (SE)**

(74) Representative: **Wu, Ping**
**Cytiva**
**Björkgatan 30**
**751 84 Uppsala (SE)**

(56) References cited:
**EP-A1- 2 578 286       WO-A1-2008/153472**
**WO-A1-2010/151214    WO-A2-93/07168**
**US-A- 5 457 260         US-B1- 6 395 538**

**Description**

TECHNICAL FIELD

The present invention relates to a method for controlling a bioprocess purification system comprising in a continuous chromatography system configured to operate with at least three columns adapted for cyclic use for continuous purification of a sample comprising a target product when feeding the continuous chromatography system with the sample.

BACKGROUND

An important factor in process chromatography is binding capacity of a chromatography column for the solute. The binding capacity directly influences the productivity and cost of the chromatography step. The binding capacity is defined either in terms of dynamic/breakthrough capacity or as the maximum binding capacity. The dynamic capacity depends on the conditions at which the solution flows through a column packed with chromatography medium, and may be represented as a ratio between column volume and feed flow rate, a so called residence time. The maximum binding capacity represents a breakthrough capacity of the column if the residence time was infinitely long.

An initial breakthrough capacity is defined as the amount of binding solutes taken up by a column at the point when the solutes are first detected in the effluent. The breakthrough capacity can also be defined as a capacity at a given percentage of breakthrough, where the percentage represents the amount of binding solute present in the effluent from the column, expressed in percent of the solute present in the feed. According to this definition the maximum binding capacity will be equal to breakthrough capacity at 100% of breakthrough, i.e., at the point where no more solute can bind to the column. Therefore, in order to determine maximum capacity, the breakthrough capacities are measured at different levels of breakthrough, where the levels are defined by levels of concentration of solutes measured in the effluent from the column during sample loading.

Often these concentrations are determined by continuously monitoring a signal in a flow through a detector placed in the effluent line. The plot of these concentrations (signal) against time (or volume or mass loaded) is called a breakthrough curve. Location of the breakthrough on a chromatogram and its shape is related to how much solute the column is capable of binding and how quickly all adsorption sites are saturated with the solute. It also shows how much more solute can be bound to the column at any given time.

Breakthrough binding capacity for the solute is, in the presence of the impurities, one of the most critical parameters to optimize when developing a purification protocol. Because impurities often have similar light adsorbing properties as the solute determination of binding breakthrough capacities is a tedious and laborious work. In a typical experiment effluent from the column is collected in series of fraction, which are subsequently analysed using high resolution analysis techniques for the product in question, such HPLC, biological assays, ELISA, mass spectrometry, etc. Thus the determination of binding capacities for a chromatography column is rather complicated and in cases where the feed solution concentration is randomly varying during the feed application onto a chromatography column the true breakthrough capacities are close to impossible to measure accurately.

Accurate measuring is important if one wants to operate a column at the optimum process conditions. For instance, it can be shown that under certain conditions a maximum productivity of a capture chromatography step is obtained when the solute of interest reaches a certain value of its concentration in the column effluent, for instance a 10% of its initial concentration. If the breakthrough capacity is determined according to the method described above, it is impossible to terminate loading of the column at exact 10% breakthrough if either feed concentration or process conditions, including flow rate and/or chromatography media properties, vary with time in unpredictable manner.

Furthermore, accurate determination of breakthrough capacities at different levels of breakthrough under varying process conditions is also important in the case of continuous chromatography. Continuous chromatography can be realised by a system operating using simulated moving bed technology, wherein the connections between the columns is changed to facilitate a continuous feed of sample into the system. However, continuous chromatography may also be realised using moving bed technologies, wherein the columns are moved to facilitate continuous feed of sample.

In continuous chromatography, several identical, or almost identical, columns are connected in an arrangement that allows columns to be operated in series and/or in parallel, depending on the method requirements. Thus, all columns can be run in principle simultaneously, but with slightly shifted method steps. The procedure can be repeated, so that each column is loaded, eluted, and regenerated several times in the process. Compared to 'conventional' chromatography, wherein a single chromatography cycle is based on several consecutive steps, such as: load the sample, wash, elution, strip, Clean-In-Place (CIP) and re-equilibration, before the column may be used for another batch, in continuous chromatography based on multiple identical columns all these steps occur simultaneously but on different columns each.

Continuous chromatography operation results in better utilization of chromatography resin, reduced processing time and reduced buffer requirements, all of which benefits process economy.

Continuous chromatography is an example of periodic counter current process, because periodically all the chromatography columns comprising the system are simultaneously moved in the direction opposite to the sample flow. The apparent movement of the columns is realized by appropriate redirections of inlet and outlet stream to/from the columns.

Historically, essential factors for a reliable continuous process are:

1) the quality of the columns used, and more specifically the similarity or even identity between columns,
2) constant feed composition, and
3) hardware reliance, for instance constant flow rate delivered by pumps, valve functionality, etc.

If the columns are not identical, the theoretical calculations typically used to design continuous chromatography process will not be correct, and it will become difficult to design an efficient and robust continuous chromatography process. The same argument applies if feed concentration and flow rates vary with time in an unexpected manner.

Therefore, for scale-up considerations, having identical columns, reliable pumps in the system is essential. However, the packing of a column with a chromatography media is very complex in order to obtain repeatable results. Even small differences in the number of plates or other packing properties can have a huge effect on the end result. Furthermore, since capacities of chromatography resins typically change during resins lifetime/usage the process conditions chosen for a fresh resin may not be applicable for a resin that has been used for several times. If also the feed solution concentration will vary it will be even more complicated to design an efficient continuous chromatography process that would operate at its optimum all the time.

An example of continuous chromatography, configured to operate with three or four columns, is ÄKTA™pcc 75 produced by GE Health Care (description available from www.gelifesciences.com/AKTA).

The process performance of the ÄKTA™pcc 75 is monitored through trend curves for UV, amount of target molecules in elution peaks, and sample volume loaded on each column per cycle.

WO2010151214 discloses a method for determining binding capacities of a chromatography column comprising: - detecting a feed signal representative of the composition of a feed material provided to the inlet of the column; - detecting an effluent signal representative of the composition of the effluent from the column; - using the feed signal and the effluent signal to determine binding capacities of the column. And a method for controlling a chromatography system comprising at least one column, comprising the steps of: - determining binding capacities of the at least one chromatography column according to above; and - controlling the start and stop of the different chromatography process steps according to the determined binding capacities.

However, since a bioprocess purification system is intended to run for a long duration (often more than thirty days) it is essential that the product produced by the system maintain the same characteristics during the whole duration..

Thus, there is a need to introduce a process for controlling the characteristics of the target product continuously to identify deviations that may affect the characteristics of the target product in real time.

SUMMARY

An object of the present disclosure is to provide methods and devices configured to execute methods and computer programs which seek to mitigate, alleviate, or eliminate one or more of the above-identified deficiencies in the art and disadvantages singly or in any combination.

The object is achieved by a method for controlling a bioprocess purification system, a bioprocess purification system, a computer program and a computer-readable storage medium as defined in claims 1-17.

An advantage is that the quality of the target product is increased.

Another advantage is that the continuous chromatography is more efficiently used in the bioprocess purification system.

Further objects and advantages may be obtained from the detailed description by a skilled person in the art.

BRIEF DESCRIPTION OF THE DRAWINGS

Fig. 1 illustrates an overview of a bioprocess purification system designed to purify a target product using continuous chromatography.

Fig. 2 illustrates a continuous chromatography with an arbitrary number of columns, based on a simulated moving bed technology.

Figs. 3a-3c illustrate the principle of three column chromatography.

Figs. 4a-4d illustrate capacity utilization for conventional batch chromatography operation compared to multi-column chromatography operation.

Fig. 5 illustrates an overview of two-step breakthrough in continuous chromatography.

Fig. 6 illustrates UV signal detectors used for dynamic control in continuous chromatography.

Fig. 7a illustrates the concept of controlling upstream/downstream processes in a bioprocess purification system.

Fig. 7b illustrates data used for trend analysis.

Fig. 8 illustrates a process for controlling characteristics of a target product in bioprocess purification system.

Fig. 9 illustrates a graphical interface for a continuous chromatography in a first occasion.

Figs. 10a-10d illustrate graphs for monitoring operational status of columns in a continuous chromatography.

Fig. 11 illustrates a graphical interface for a continuous chromatography in a second occasion.

Fig. 12 illustrates a real time evaluation interface.

Fig. 13 illustrates load volume data used for trend analysis.

DETAILED DESCRIPTION

A continuous chromatography is designed for purification of target products (such as proteins, biomolecules from cell culture/fermentation, natural extracts) in continuous downstream processes using periodic counter current chromatography, as explained in background section. The technology employs three or four chromatography columns to create a continuous purification step. The columns are switched between loading and non-loading steps, such as wash and elution. Continuous chromatography supports process intensification by reducing footprint and improving productivity. In addition, continuous chromatography is especially suited for purification of unstable molecules, as the short process time helps to ensure stability of the target product.

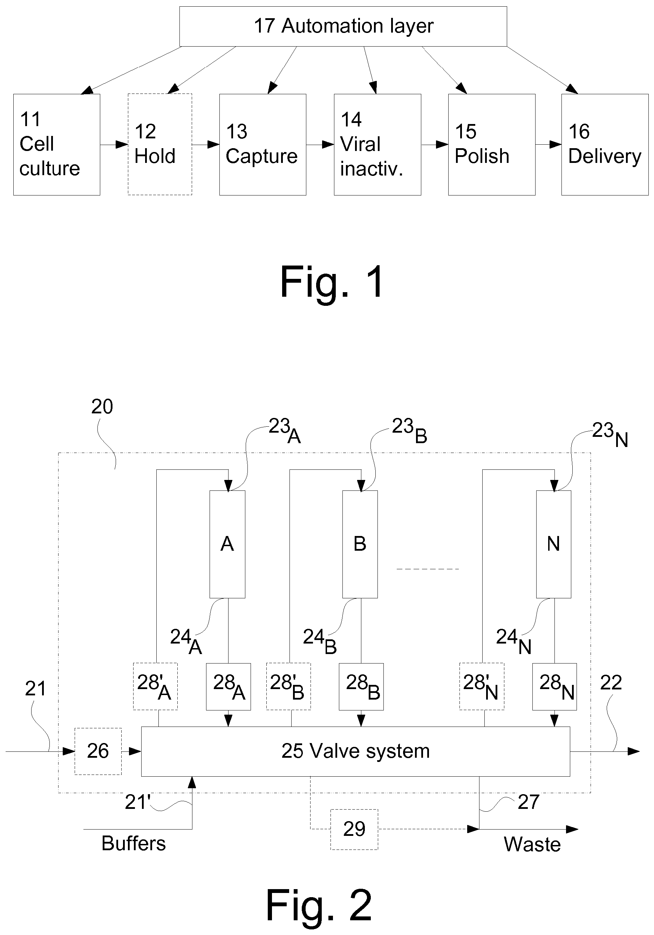

In figure 1, an overview of a bioprocess purification system, configured to purify a target product using a separation process is shown. The bioprocess purification system comprises a number of steps related to Cell culture 11, Hold 12, Capture 13, Viral inactivation 14, Polish 15 and Delivery 16.

In a fully continuous process the cell culture step 11 may be a perfusion type culture which comprises continuous addition of nutrients for cell growth in perfusion culture and continuous removal of product and waste through drain and filtration. E.g. using an Alternate Tangential Filtration( ATF) filter. The step may comprise process control for viable cell density (VCD), and the next step in the process starts when VCD reaches a pre-determined value. The VDC may be controlled by adapting the components of the cell culture media fed to the culture or by addition of certain components directly to the culture. Alternatively, the cell culture is of batch type.

The sample containing the target product is exploited in a cell free extraction process, e.g. by filtration, centrifugation or another technique.

The hold step 12 is an optional step depending on process needs, e.g. if a filter is in-line before capture step 13. The step may comprise process control on weight, and the next step in the process starts when a pre-determined volume value is reached, or alternatively after a certain time period or when a pre-determined mass is reached. The hold step may be used both for collecting a volume of filtered feed from a perfusion cell culture or from a batch culture.

The capture step 13 comprises a continuous chromatography that may have a filter in-line before the capture step. The continuous chromatography may be run as periodic counter current chromatography with a continuous feed of sample from the cell culture step 11, directly or via the hold step 12, containing the target product. The capture step comprises multiple batch elutions, and process control using in-line UV-sensors handles variation in feed concentration and resin capacity. The next step starts when a pre-determined amount value (e.g. volume, mass or time) is reached.

In the viral inactivation step 14, different options for virus inactivation is available depending on process needs. One option is to use batch mode with low pH for 30-60 minutes in hold up tank. The step may comprise process control on volume, time, temperature and pH. The next step starts when a pre-determined time is reached.

The polish step 15 may be straight through processing (STP) with a connected batch step or continuous chromatography with a continuous load step, or a combination thereof. The flow rate is adjusted to perfusion rate required by producer cells, which means that the flow rate is determined by the preceding step. The step may comprise process control for UV, flow and volume, and the next step starts when a pre-determined volume and amount is reached, alternatively when a timeout is reached.

The delivery step 16 may comprise a virus removal step, e.g. a viral filter, before an ultrafiltration step. The delivery step may be used as concentration step for batch addition of sample from polish step. The delivery step may comprise continuous or batch delivery of product and may comprise continuous or batch removal of waste. The step may comprise process control for pH, conductivity, absorbance, volume and pressure, and delivery is achieved when a pre-determined product concentration in a pre-defined environment is reached.

An automation layer 17 is used for handling decision points for next step in the process. Different type of sensors (not shown), both in-line sensors and off-line sensors, are integrated into the process flow to monitor different parameters that may be used for providing the automation layer 17 with data that could be used to handle the decision points. Sensors include but are not limited to only measure flow, VCD, weight, pressure, UV, volume, pH, conductivity, absorbance, etc.

It should be noted that UV an example of a parameter that could be monitored to detect the composition of the sample being purified. However, other parameters may be used operating in other frequency ranges, such as IR, fluorescence, x-rays, etc.

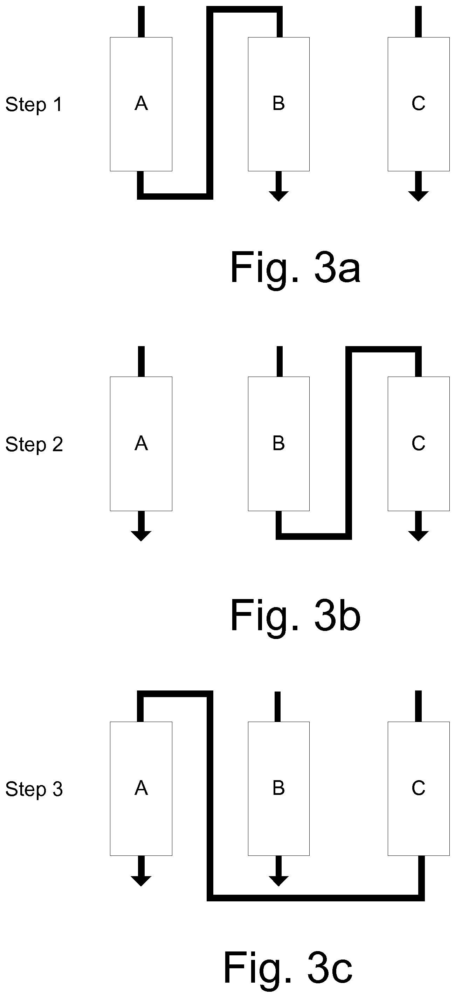

The capture step 13 may comprise a continuous chromatography 20, as illustrated in figure 2. Sample containing the target product is fed into the continuous chromatography 20 via inlet 21 and the eluted target product is available at outlet 22. The continuous chromatography 20 comprises multiple columns A, B,...,N, and each column is provided with a column inlet 23 and column outlet 24. The column inlet 23 and column outlet 24 of each column is connected to a valve system 25 configured to connect the columns cyclically to the inlet 21 and the outlet 22 to achieve continuous purification of the target product. Example of a system configuration having three columns is described in connection with figure 3a-3c.

The continuous chromatography 20 is further provided with buffer inlet 26 and waste outlet 27 in order to be able to perform the required operations. An in-line sensor 28 may provide after the column outlet 24 of each column or be assigned to the process flow and integrated into the valve system 25. Important parameters, such as UV, is measured to control the process, as described below. Another in-line sensor 28' may be provided before the column inlet 23 of each column in order to be able to directly evaluate performance of each column. An in-line inlet sensor 26 may also be provided to monitor the composition of the sample fed into the continuous chromatography 20

The continuous chromatography may also comprise off-line sensors 29, which are designed to extract material from the process and thereafter evaluate selected parameters before the material is disposed of as waste.

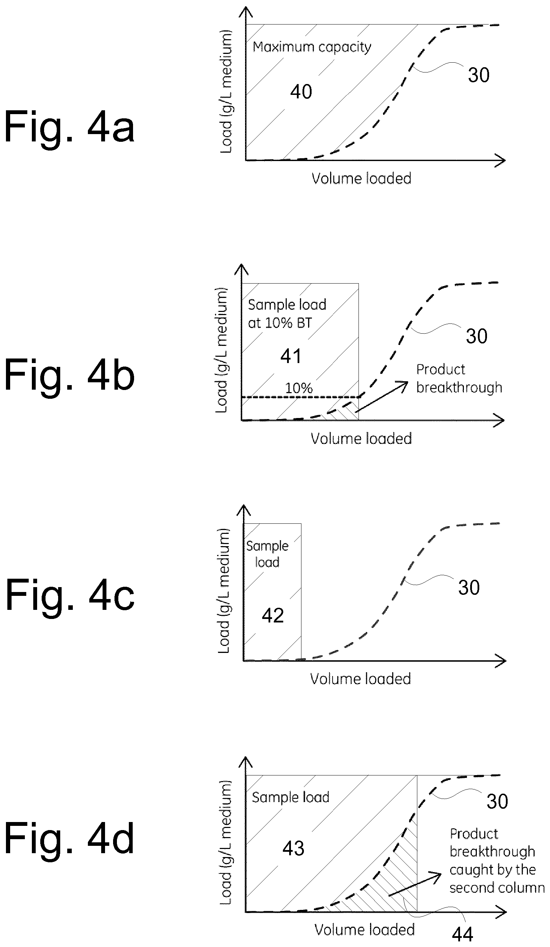

The principle behind the continuous chromatography is to keep at least two columns in the loading zone which allows for overloading of the first column without risk of product loss, as the breakthrough will be caught by downstream columns, as described in connection with figures 4a-4d.

The continuous chromatography comprises at least three columns and the principle of operations in a three columns (3C) setup is described in connection with figures 3a-3c. The 3C setup features two parallel flows: one for loading of the two columns in the loading zone, and one for the non-loading steps, e.g. elution and regeneration of the third column.

In figure 3a, illustrating step 1, column A and B are in the loading zone. Column A can be overloaded without sample loss, as column B catches the breakthrough from column A. In this way, the utilization of the resin binding capacity is maximized.

In figure 3b, illustrating step 2, the overloaded column A is switched and column B becomes the first column and column C becomes the second column in the loading zone. The overloaded column A will now be subjected to the non-loading steps, such as elution and regeneration in a parallel workflow.

In figure 3c, illustrating step 3, the overloaded column B in the loading zone is switched. Now column C becomes the first column and column A the second column in the loading zone, while column B is subjected to elution and regeneration in the parallel workflow. These three steps are repeated in a cyclic manner until required target product volume, mass or amount is reached (or until resin lifetime is reached and columns needs to be repacked or exchanged).

The continuous chromatography illustrated in figure 2 may utilize more than three columns, and in a four column (4C) setup, the same principle applies. However, the non-loading steps can become limiting in a 3C setup, and the non-loading steps can be split on two columns and run in parallel utilizing a third flow path in the 4C setup. The 4C setup allows for balancing the loading and non-loading steps. More columns will lead to a more flexible system, while the complexity of the valve system 25 becomes increasingly complicated. However, some continuous chromatography have sixteen or more columns.

Figures 4a-4b illustrate capacity utilization for conventional batch chromatography operation compared to multi-column chromatography operation. Figure 4a illustrates the total available capacity 40 of a chromatography resin and breakthrough curve is indicated by 30. As is apparent from the graph, when a small volume is loaded the product will be captured in the resin, but at large volumes a substantial part of the product will breakthrough and be wasted in a batch operation.

Figure 4b illustrates the sample load 41 at 10% breakthrough. The product below the breakthrough curve 30 will be wasted if not reused in another column. The capacity 42 typically used in batch operations is illustrated in Figure 4c, and capacity 43 used in continuous chromatography is illustrated in figure 4d. Note that the product breakthrough 44 is captured by the columns downstream in the loading zone.

Dynamic control functionality, which allows for some variations in feed composition, may be implemented in the continuous chromatography. The principle of dynamic control is based on the relative difference in UV signals before and after each column at breakthrough. The difference between the baseline UV and the UV signal at 100% breakthrough for a saturated column is defined as $\Delta UV_{max}$, wherein $\Delta UV$ is calculated using equation (1)

$$\Delta UV = 100 \times \frac{UV_{BT} - Baseline}{UV_{sample} - Baseline} \tag{1}$$

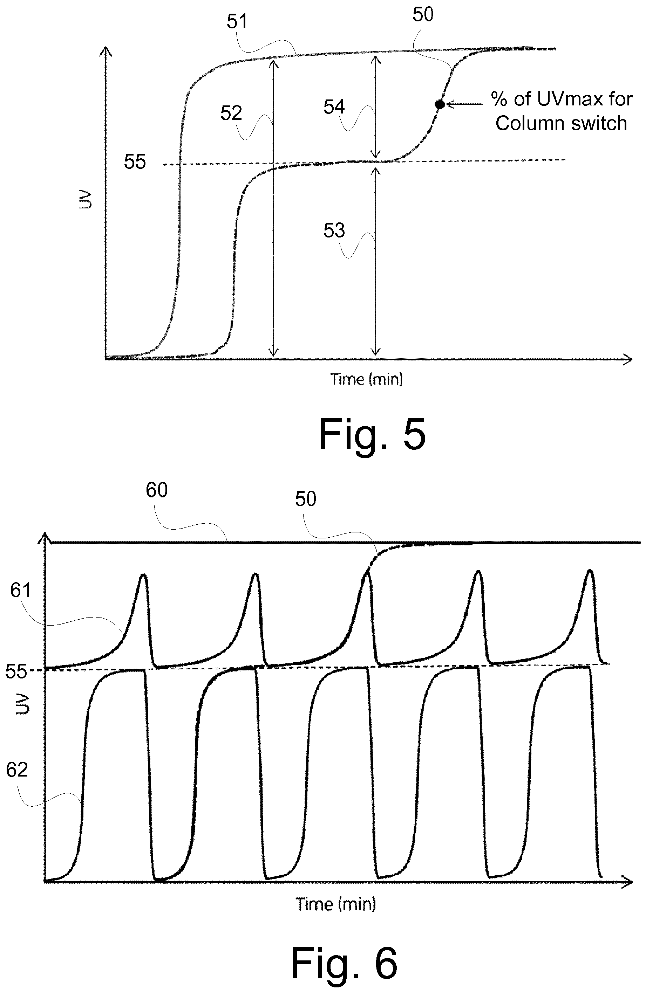

Figure 5 illustrates the two-step breakthrough, displaying the central UV signals used for dynamic control. Curve 50 shows the $UV_{BT}$ (i.e. the post column breakthrough curve), curve 51 shows the $UV_{sample}$ (i.e. the pre column feed line), and reference numeral 55 indicates the baseline. Reference numeral 52 indicates total signal from target product and impurities in the sample being fed into the column, reference numeral 53 indicates signal from impurities (background), and reference numeral 54 indicates $\Delta UV_{max}$ (signal from target product).

The difference between the baseline UV 55 and the UV signal at 100% breakthrough for a fully loaded column is defined as $\Delta UV_{max}$, where the desired level is process-dependent. A continuous chromatography may use UV detectors assigned to the process stream and not to the separate columns. Hence, each breakthrough curve may be generated based on signals from two UV detectors as illustrated in figure 6.

The breakthrough curve (dashed curve 50) and the baseline 55 are the same as shown in figure 5. Curve 60 is UV of the sample fed into the column, curve 61 is UV of the breakthrough after the column and curve 62 is UV of the flow through (waste).

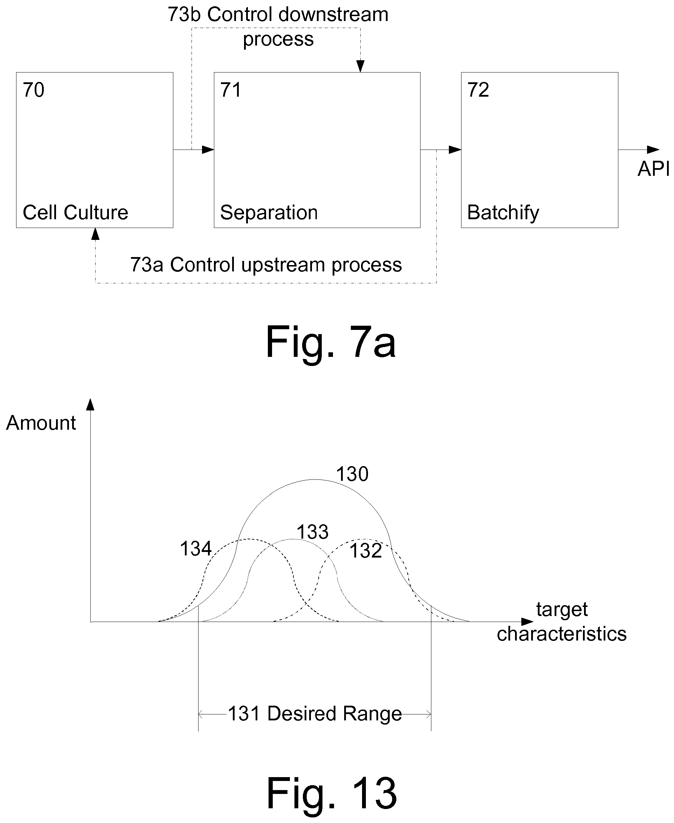

Figure 7a illustrates the concept of controlling upstream/downstream processes in a bioprocess purification system. The illustration of the bioprocess purification system is simplified and comprises three steps: Cell culture 70, Separation 71 and batchify 72. The target product (in this example exemplified by "active pharmaceutical ingredient" - API) is delivered after the batchify step.

The Cell culture step includes continuous addition of nutrients to a cell perfusion process with continuous removal of target product and waste. The target product and waste is considered to be the sample that is fed into the Separation step 71. The separation step comprises a process for separating the target product from the waste in the sample and the target product is forwarded to the final step Batchify 72, in which the target product is handled to be ready for delivery as API.

After the separation step, certain parameters may be measured, e.g. composition of impurities in target product or amount of broken molecules of the target product using mass spectrometer or spectrometry. This information may be used to control an upstream process 73a. For instance, if a high amount of broken molecules of the target product is detected after separation, this may be rectified by changing parameters in the cell culture step, such as increase flow in the cell perfusion to prevent breakdown of target product molecules before introduced into the separation step 71. Alternatively, feeding parameters in the fermentation may be adjusted based on the measured information.

The same concept may be used to control a downstream process 73b. The concentration of target product in the sample being fed into the separation step 71 may be determined by measuring the time to load each column and the peak amount of target product after elution. This information may be used to adjust the elution based on the concentration of target product in the sample being fed into the separation step.

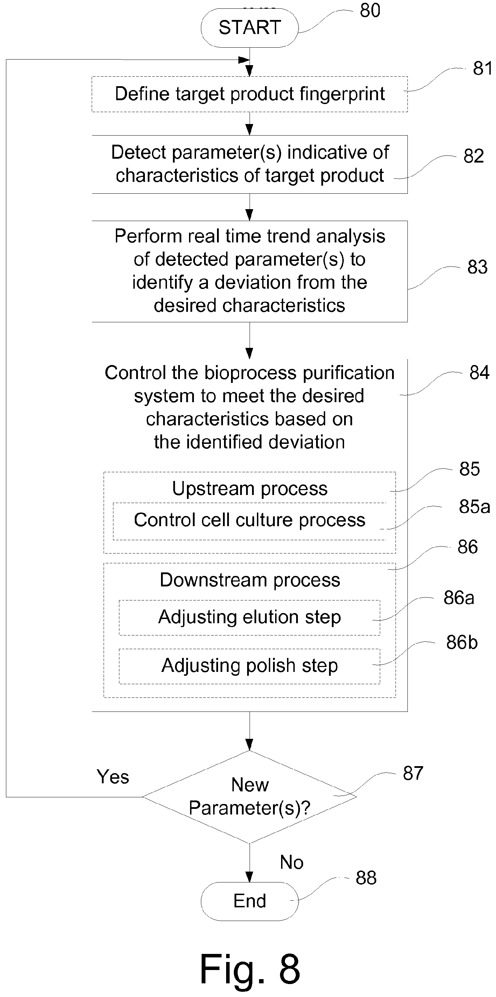

Other parameters may be used to control different processes (upstream or downstream) as disclosed more in detail in connection with figure 8.

Figure 13 illustrates real time trend analysis used to ensure desired target characteristics. Curve 130 illustrates the desired distribution of a selected target characteristics, for instance impurities, concentration, virus, etc. A pre-determined range 131 is selected based on the selected target characteristics 130. In order to be able to monitor the characteristics of the target product continuously, a process is suggested in which batches is created from the continuous flow and each batch is evaluated and quality controlled to ensure that each batch fulfil the specification of the target product.

The dot-dashed curve 132 illustrates the measurements of the selected target characteristics of a first batch. The first batch is decided to be within the pre-determined range. The dotted curve 133 illustrates the measurements of the selected target characteristics of a second batch. The second batch is decided to be within the pre-determined range. The dashed curve 134 illustrates the measurements of the selected target characteristics of a third batch. The third batch is decided not to be within the pre-determined range and is thus forwarded to waste.

Figure 8 illustrates a method for controlling a bioprocess purification system comprising a continuous chromatography configured to operate with at least three columns and configured for continuous purification in a cyclic operation, wherein the continuous purification is performed on a sample comprising a target product having a desired characteristics.

The method starts in step 80 and comprises three main steps: detecting 82 at least one parameter indicative of characteristics of the target product, performing 83 real time trend analysis of each detected at least one parameter to identify a deviation from the desired characteristics of the target product, and controlling 84 a the bioprocess purification system to meet the desired characteristics based on the identified deviation, whereby the target characteristics is within a pre-determined range.

Optionally, a step to define 81 a target product fingerprint is performed before the three main steps are initiated. According to one aspect, the target product fingerprint is defined by the composition of the target product and/or detected impurities in the target product. The fingerprints may be obtained by spectrometry.

According to the invention, detecting 82 at least one parameter comprises detecting impurities in the target product after the sample has been processed in the continuous chromatography, and a process is controlled 84 by reprocessing the target product with the detected impurities.

Reprocessing comprises processing the target product with detected impurities in a different column in the continuous chromatography compared to the column used when previously processed. The bioprocess purification system may comprise a viral inactivation step after the continuous chromatography, reprocessing may comprise processing the target product with detected impurities through another viral inactivation step, which normally is not used in the bioprocess purification system.

According to one aspect, the parameters detected in 82 is purified amount of the target product and the trend analysis in 83 is performed over time.

According to one aspect, the controlling the bioprocess purification system in 84 comprises controlling an upstream process 85, and according to one aspect the control of the upstream process comprises controlling the concentration of the target product in the sample being fed into the continuous chromatography. In addition, when the bioprocess purification system comprises a cell culture process, the control of an upstream process further comprises controlling the cell culture process 85a to adjust the composition of the sample being fed into the continuous chromatography.

According to one aspect, the controlling the bioprocess purification system in 84 comprises controlling a downstream process 86. When the continuous chromatography comprises loading, elution and cleaning steps of each column, the downstream process may comprise adjusting 86a the elution step based on the concentration of target product in the sample being fed into the continuous chromatography.

Furthermore, wherein the bioprocess purification system comprises a polish step, the downstream process may comprise using the defined target product fingerprint (step 81), and the parameter obtained in step 82 comprises regularly obtaining a fingerprint after the sample has been processed in the continuous chromatography. The target product fingerprint is thereafter compared using trend analysis in step 83 with the regularly obtained fingerprint of the sample to identify deviations, and in step 84, the polish step is adjusted 86b in response to the identified deviations.

The method described in connection with figure 8 may be implemented in a bioprocess purification system comprising a continuous chromatography configured to operate with at least three columns and configured for continuous purification in a cyclic operation, wherein the continuous purification is performed on a sample comprising a target product having a desired characteristics as disclosed in connection with figures 1 and 7a.

The bioprocess purification system is configured to detect at least one parameter indicative of characteristics of the target product, perform real time trend analysis of each detected at least one parameter to identify a deviation from the desired characteristics of the target product, and control the bioprocess purification system to meet the desired characteristics based on the identified deviation, whereby the target characteristics is within a pre-determined range.

According to one aspect, a downstream process, or an upstream process, is controlled in the bioprocess purification system.

The method described above may be implemented in a computer program for controlling a bioprocess purification system. The computer program comprises instructions which, when executed on at least one processor, cause the at least one processor to carry out the method according to the different variations described in connection with figure 8. The computer program for controlling the bioprocess purification system may be stored on and carried by a computer readable storage medium.

In the following, a specific embodiment is disclosed, related to a method for controlling a bioprocess purification system for producing a target product having a desired characteristics, the bioprocess purification system comprising at least one upstream process and a continuous chromatography process. The at least one upstream process comprises a cell culture process for producing a feed (or sample) comprising the target product. The continuous chromatography process is configured to operate with at least three columns and is configured for continuous purification in a cyclic operation, wherein the continuous purification is performed on the feed to separate the target product from other components of the feed, wherein the method comprising:

a) detecting, after purification of the target product, at least one parameter indicative of characteristics of the target product, and/or after removal of the target product from the feed at least one parameter indicative of the characteristics of the feed (for instance amount of impurities),
b) controlling at least one upstream process in response to the detected at least one parameter to produce the target product having said desired characteristics.

All information that may be linked to cell culture conditions can be used to loop back system controlled measures such as pH adjustment or addition or reduction of the carbon source, vitamins, trace elements, etc. In particular, a cell culture simulation model may be used to predict what output is expected from a given action.

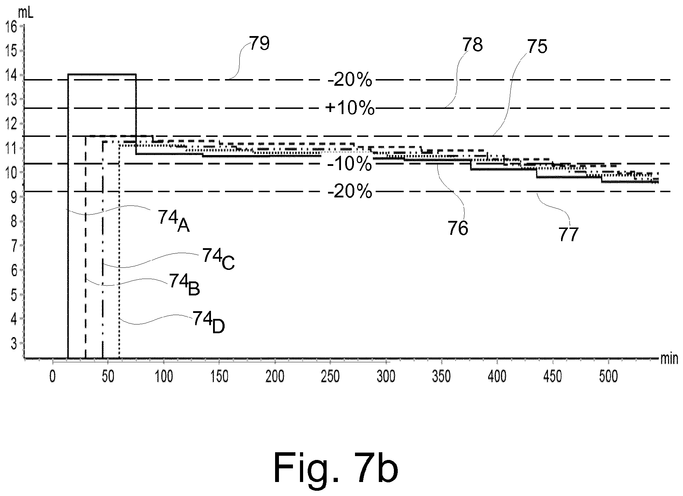

This embodiment is exemplified in figure 7b is a graph illustrating loading volume of sample (y axis) into each column A-D over run time (x axis). The solid line $74_A$ is the measured loading volume for column A, and the curve is amount is adjusted in steps approximately every 60 minutes. The same applies for column B (dashed line $74_B$), column C (double dot-dashed line $74_C$) and column D (dotted line $74_D$).

Different boundary conditions (lines 75-79) are indicated in the graph, which may be used to identify trends that indicate a deviating behaviour. If, for instance, the loading volume is more than 20% higher than the expected level (as indicated by 75) immediate action is required to maintain proper functionality in the system. This is illustrated for column A, which exceeds the +20% level (as indicated by 79) after the first cycle. This may be an indication that the concentration of target product in the sample fed into the column is too low. However, this is only shown for column A during the first cycle and may be a result of the start-up process.

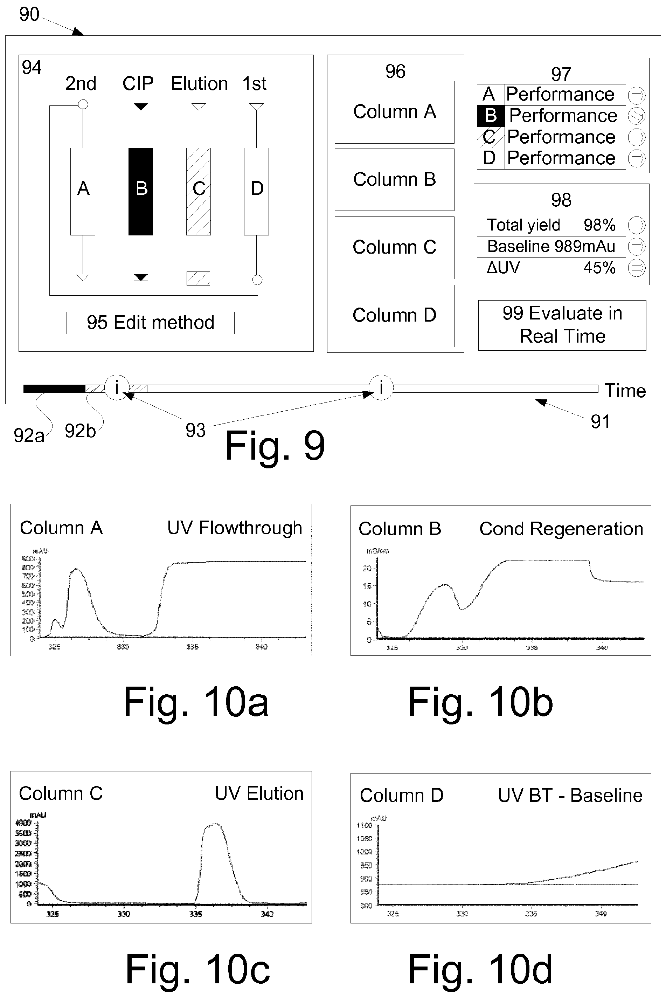

Apart from the first cycle for column A, all curves $74_A$-$74_D$ show the same behaviour, which is a steady declining behaviour and after 7-8 cycles, the lower warning level (as indicated by 76) is passed and this indicate that the concentration of target product in the sample is increasing. Action may be needed to ensure proper functionality. Figure 9 illustrates a graphical interface 90 for a continuous chromatography at a first occasion. The graphical interface is divided into several areas having different purposes. In the lower part of the graphical interface, a time line 91 is used to illustrate the projected run time, the executed run time 92a of the process, and the planned process 92b. Information pointers are illustrated to highlight certain events 93 that needs to be handled during the projected run time, such as scheduled maintenance, to prevent unscheduled downtime of the production.

The main area comprises an overview 94 of the chromatography process illustrating the process of each column A-D of the continuous chromatography. At the first occasion, column D (denoted 1st) is fed with sample from the cell culture process and the outlet of column D is connected with the inlet of column A (denoted 2nd). This means that column D and A are in the loading phase. The loaded column C (denoted Elution) is eluted and the outlet of column C is treated in subsequent steps in the bioprocess purification system. Column B (denoted CIP) was previously eluted and is cleaned to be able to be loaded with sample in a the future. The overview also include a possibility to adjust the purification method manually as indicated by 95. However, the planned process 92b is preferably not altered since this may influence the uptime of the system.

The main screen also comprises an area 96 illustrating operational status of the columns A-D, which are described in more detail in connection with figures 10a-10d. Performance of each column A-D is shown in 97, indicating where in the process each column is and also a real time trend indication. in this case, the performance of column B is highlighted and action is needed to ensure proper functionality. This may be planned and is the reason for the maintenance scheduled in the near future, as indicated by the first information pointer 93.

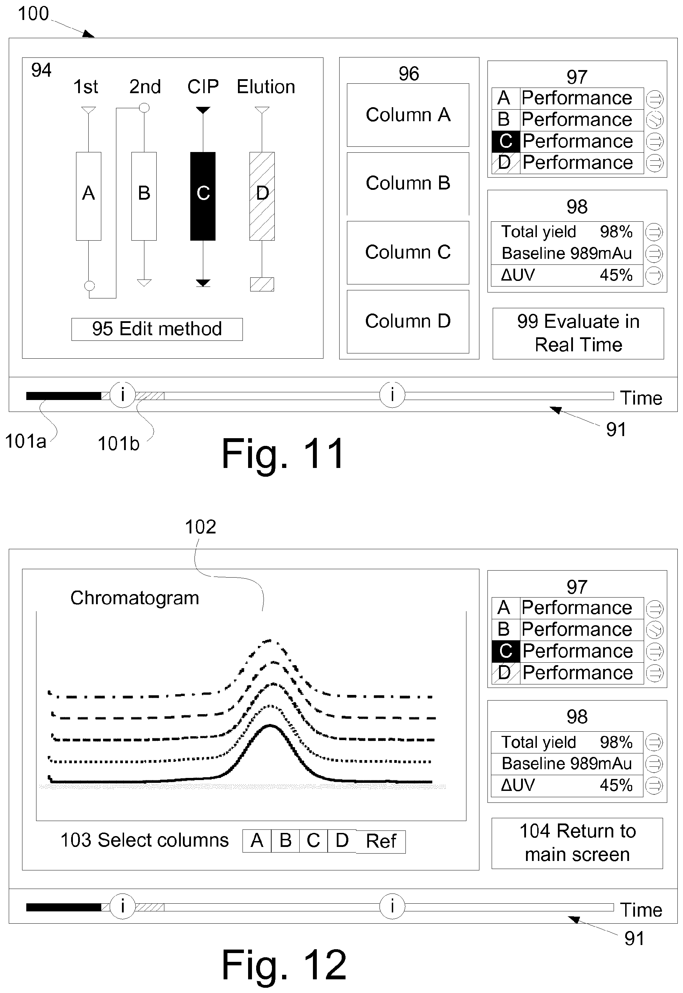

Certain key data, which are important for the process are presented in a separate area 98. Examples of key data is Total yield, Baseline, ΔUV. In order to be able to monitor and evaluate in real time, a button 99 is provided to switch between trend curves and the main screen. An example of an evaluation interface is shown in connection with figure 12.

Figures 10a-10d illustrate graphs for monitoring operational status of columns A-D in a continuous chromatography. Figure 10a illustrate the UV Flow through of column A, figure 10b illustrate the conductivity during regeneration for column B, figure 10c illustrate the UV Elution for column C and figure 10d illustrate the UV breakthrough - baseline for column D.

Fig. 11 illustrates a graphical interface 100 for a continuous chromatography at a second occasion. The graphical interface 100 is divided into the same areas as described in figure 9.

However, the time line 91 is adjusted to compensate for the time elapsed between the first occasion and the second occasion. The time line 91 is used to illustrate the projected run time, the executed run time 101a has been adjusted as well as the planned process 101b.

The overview 94 of the chromatography process illustrating the process of each column A-D of the continuous chromatography is updated. At the second occasion, column A (denoted 1st) is fed with sample from the cell culture process and the outlet of column A is connected with the inlet of column B (denoted 2nd). This means that column A and B are in the loading phase. The loaded column D (denoted Elution) is eluted and the outlet of column D is treated in subsequent steps in the bioprocess purification system. Column C (denoted CIP) was previously eluted and is cleaned to be able to be loaded with sample in a the future.

Figure 12 illustrates a real time evaluation interface, which is activated when an operator press the evaluation button 99, as described in connection with figure 9. The most important areas are still available in the evaluation interface, i.e. time line 91, performance 97 and key data 98. The major area of the evaluation interface shows a chromatogram 102 illustrating comparisons between selected parameters detected by sensors in the continuous chromatography. Different columns may be selected, as indicated by 103.

To return to the main screen a button 104 is provided for the user.

## Claims

1. A method for controlling a continuous bioprocess purification system comprising a continuous chromatography (13;20) configured to operate with at least three columns (A;B;C;N) and configured for continuous purification in a cyclic operation, wherein the continuous purification is performed on a sample comprising a target product having a desired characteristics, **characterized in that** the method comprises:

   a) detecting (82) at least one parameter indicative of characteristics of the target product,
   b) and controlling (84) the bioprocess purification system to meet the desired characteristics based on the detected at least one parameter,
   whereby the desired characteristics is within a pre-determined range,
   wherein the continuous chromatography is run as a periodic counter current chromatography,
   wherein impurities in the target product is detected in step a) after the sample has been processed in the continuous chromatography (13;20), and step b) comprises reprocessing the target product with the detected impurities, and
   wherein the reprocessing of the target product comprises processing the target product with the detected impurities in a different column (A;B;C;N) in the continuous chromatography (13;20) compared to the column (A;B;C;N) used when previously processed.

2. The method according to claim 1, wherein the bioprocess purification system comprises a viral inactivation step (14) after the continuous chromatography (13;20) and the reprocessing of target product with detected impurities comprises processing through another viral inactivation step (14).

3. The method according to any of claims 1-2, wherein step b) controls an upstream process (85) in the bioprocess purification system.

4. The method according to claim 3, wherein step b) comprises controlling the concentration of the target product in the sample being fed into the continuous chromatography (13;20).

5. The method according to claim 3, wherein the bioprocess purification system comprises a cell culture process (11) and step b) comprises controlling (85a) the cell culture process (11) to adjust the composition of the sample being fed into the continuous chromatography (13;20).

6. The method according to any of claims 1-2, wherein step b) controls a downstream process (86) in the bioprocess purification system.

7. The method according to claim 6, wherein the continuous chromatography (13, 20) comprises loading, elution and cleaning steps of each column (A;B;C;N) and step b) comprises adjusting (86a) the elution step based on the concentration of target product in the sample being fed into the continuous chromatography (13;20).

8. The method according to claim 6, wherein the bioprocess purification system comprises a polish step (15) and wherein the method further comprises:

   - defining (81) a target product fingerprint,
   - in step a), regularly obtaining a fingerprint after the sample has been processed in the continuous chromatography (13;20), and comparing the target product fingerprint with the regularly obtained fingerprint of the sample to identify deviations, and
   - in step b), adjusting (86b) the polish step (15) in response to the identified deviations.

9. The method according to claim 8, wherein the target product fingerprint is defined by the composition of the target product and/or detected impurities in the target product.

10. The method according to claim 8 or 9, wherein the fingerprints are obtained by spectrometry.

11. The method according to any of claims 1-10, wherein the method further comprises performing (83) real time trend analysis of each of the detected at least one parameter to identify a deviation from the desired characteristics of the target product before step b), and controlling the bioprocess purification system based on the identified deviation.

12. The method according to claim 11, wherein purified amount of the target product is measured in step a), and the trend analysis is performed over time.

13. A bioprocess purification system comprising a continuous chromatography (13;20) configured to operate as a periodic counter current chromatography with at least three columns (A;B;C;N) and configured for continuous purification in a cyclic operation, wherein the continuous purification is performed on a sample comprising a target product having desired characteristics, wherein the bioprocess purification system is further configured to:

- detect (82) at least one parameter indicative of characteristics of the target product,
- control (84) the bioprocess purification system to meet the desired characteristics based on the detected at least one parameter,
whereby the desired characteristics is within a pre-determined range,
wherein the detecting (82) further comprises detecting impurities in the target product after the sample has been processed in the continuous chromatography (13;20), and the control (84) further comprises reprocessing the target product with the detected impurities, and
wherein the reprocessing of the target product comprises processing the target product with the detected impurities in a different column (A;B;C;N) in the continuous chromatography (13;20) compared to the column (A;B;C;N) used when previously processed.

14. The bioprocess purification system according to claim 13, further configured to perform real time trend analysis of each detected at least one parameter to identify a deviation from the desired characteristics of the target product, and the control of the bioprocess purification system is further based on the identified deviations.

15. The bioprocess purification system according to claim 13 or 14, wherein a downstream process, or an upstream process, is controlled in the bioprocess purification system.

16. A computer program for controlling a bioprocess purification system, comprising instructions which, when executed on at least one processor, cause the at least one processor to carry out the method according to any of claims 1-12.

17. A computer-readable storage medium carrying a computer program for controlling a bioprocess purification system according to claim 16.

**Patentansprüche**

1. Verfahren zur Steuerung eines kontinuierlichen Bioprozess-Reinigungssystems, das eine kontinuierliche Chromatographie (13; 20) umfasst, die so konfiguriert ist, dass sie mit mindestens drei Säulen (A; B; C; N) arbeitet und für eine kontinuierliche Reinigung in einem zyklischen Betrieb konfiguriert ist, wobei die kontinuierliche Reinigung an einer Probe durchgeführt wird, die ein Zielprodukt mit einer gewünschten Eigenschaft umfasst, **dadurch gekennzeichnet, dass** das Verfahren Folgendes umfasst:

a) Erfassen (82) mindestens eines Parameters, der die Eigenschaften des Zielprodukts angibt,
b) und Steuern (84) des Bioprozess-Reinigungssystems, um die gewünschten Eigenschaften auf der Grundlage des erfassten mindestens einen Parameters zu erfüllen,
wobei die Zieleigenschaften innerhalb eines vorgegebenen Bereichs liegen, wobei die kontinuierliche Chromatographie als eine periodische Gegenstromchromatographie durchgeführt wird,
wobei Verunreinigungen in dem Zielprodukt in Schritt a) erfasst werden, nachdem die Probe in der kontinuierlichen Chromatographie (13; 20) verarbeitet wurde, und Schritt b) die erneute Verarbeitung des Zielprodukts mit den erfassten Verunreinigungen umfasst, und
wobei die erneute Verarbeitung des Zielprodukts die Verarbeitung des Zielprodukts mit den erfassten Verunreinigungen in einer anderen Säule (A; B; C; N) in der kontinuierlichen Chromatographie (13; 20) im Vergleich zu der Säule (A; B; C; N), die bei der vorherigen Verarbeitung verwendet wurde, umfasst.

2. Verfahren nach Anspruch 1, wobei das Bioprozess-Reinigungssystem einen viralen Inaktivierungsschritt (14) nach der kontinuierlichen Chromatographie (13; 20) umfasst und die erneute Verarbeitung des Zielprodukts mit erfassten Verunreinigungen die Verarbeitung durch einen anderen viralen Inaktivierungsschritt (14) umfasst.

3. Verfahren nach einem der Ansprüche 1-2, wobei Schritt b) einen vorgeschalteten Prozess (85) in dem Bioprozess-Reinigungssystem steuert.

4. Verfahren nach Anspruch 3, wobei Schritt b) die Kontrolle der Konzentration des Zielprodukts in der Probe umfasst, die in die kontinuierliche Chromatographie (13; 20) eingespeist wird.

5. Verfahren nach Anspruch 3, wobei das Bioprozess-Reinigungssystem einen Zellkulturprozess (11) umfasst und Schritt b) die Steuerung (85a) des Zellkulturprozesses (11) umfasst, um die Zusammensetzung der in die kontinuierliche Chromatographie (13; 20) eingespeisten Probe einzustellen.

6. Verfahren nach einem der Ansprüche 1-2, wobei Schritt b) einen nachgeschalteten Prozess (86) in dem Bioprozess-Reinigungssystem steuert.

7. Verfahren nach Anspruch 6, wobei die kontinuierliche Chromatographie (13, 20) Lade-, Elutions- und Reinigungsschritte jeder Säule (A; B; C; N) umfasst und Schritt b) das Einstellen (86a) des Elutionsschritts auf der Grundlage der Konzentration des Zielprodukts in der in die kontinuierliche Chromatographie (13; 20) eingespeisten Probe umfasst.

8. Verfahren nach Anspruch 6, wobei das Bioprozess-Reinigungssystem eine Polierstufe (15) umfasst und wobei das Verfahren weiter umfasst:

   - Definieren (81) eines Zielprodukt-Fingerabdrucks,
   - in Schritt a) regelmäßiges Erhalten eines Fingerabdrucks, nachdem die Probe in der kontinuierlichen Chromatographie (13; 20) verarbeitet wurde, und Vergleichen des Zielprodukt-Fingerabdrucks mit dem regelmäßig erhaltenen Fingerabdruck der Probe, um Abweichungen zu identifizieren, und
   - in Schritt b) Anpassen (86b) der Polierstufe (15) als Reaktion auf die identifizierten Abweichungen.

9. Verfahren nach Anspruch 8, wobei der Zielprodukt-Fingerabdruck durch die Zusammensetzung des Zielprodukts und/oder in dem Zielprodukt erfasste Verunreinigungen definiert ist.

10. Verfahren nach Anspruch 8 oder 9, wobei die Fingerabdrücke durch Spektrometrie erhalten werden.

11. Verfahren nach einem der Ansprüche 1-10, wobei das Verfahren weiter das Durchführen (83) einer Echtzeit-Trendanalyse jedes der erfassten mindestens einen Parameter umfasst, um eine Abweichung von den gewünschten Eigenschaften des Zielprodukts vor Schritt b) zu identifizieren, und das Steuern des Bioprozess-Reinigungssystems auf der Grundlage der identifizierten Abweichung.

12. Verfahren nach Anspruch 11, wobei die gereinigte Menge des Zielprodukts in Schritt a) gemessen wird und die Trendanalyse über die Zeit durchgeführt wird.

13. Bioprozess-Reinigungssystem, umfassend eine kontinuierliche Chromatographie (13; 20), die so konfiguriert ist, dass sie als periodische Gegenstromchromatographie mit mindestens drei Säulen (A; B; C; N) arbeitet und für eine kontinuierliche Reinigung in einem zyklischen Betrieb konfiguriert ist, wobei die kontinuierliche Reinigung an einer Probe durchgeführt wird, die ein Zielprodukt mit gewünschten Eigenschaften umfasst, wobei das Bioprozess-Reinigungssystem weiter zu Folgendem konfiguriert ist:

   - mindestens einen Parameter zu erfassen (82), der die Eigenschaften des Zielprodukts anzeigt,
   - das Bioprozess-Reinigungssystem so zu steuern (84), dass es die gewünschten Eigenschaften auf der Grundlage des erfassten mindestens einen Parameters erfüllt,
   wobei die Zieleigenschaften innerhalb eines vorgegebenen Bereichs liegen,
   wobei das Erfassen (82) weiter das Erfassen von Verunreinigungen in dem Zielprodukt umfasst, nachdem die Probe in der kontinuierlichen Chromatographie (13; 20) verarbeitet worden ist, und das Steuern (84) weiter das erneute Verarbeiten des Zielprodukts mit den erfassten Verunreinigungen umfasst, und
   wobei die erneute Verarbeitung des Zielprodukts die Verarbeitung des Zielprodukts mit den erfassten Verunreinigungen in einer anderen Säule (A; B; C; N) in der kontinuierlichen Chromatographie (13; 20) im Vergleich zu der Säule (A; B; C; N), die bei der vorherigen Verarbeitung verwendet wurde, umfasst.

14. Bioprozess-Reinigungssystem nach Anspruch 13, weiter konfiguriert, um eine Echtzeit-Trendanalyse jedes erfassten mindestens einen Parameters durchzuführen, um eine Abweichung von den gewünschten Eigenschaften des Zielprodukts zu identifizieren, und wobei das Steuern des Bioprozess-Reinigungssystems weiter auf den identifizierten Abweichungen basiert.

15. Bioprozess-Reinigungssystem nach Anspruch 13 oder 14, wobei ein nachgeschalteter Prozess oder ein vorgeschalteter Prozess in dem Bioprozess-Reinigungssystem gesteuert wird.

16. Computerprogramm zur Steuerung eines Bioprozess-Reinigungssystems, das Befehle umfasst, die, wenn sie auf mindestens einem Prozessor ausgeführt werden, den mindestens einen Prozessor veranlassen, das Verfahren nach einem der Ansprüche 1-12 auszuführen.

17. Computerlesbares Speichermedium, das ein Computerprogramm zur Steuerung eines Bioprozess-Reinigungssystems nach Anspruch 16 enthält.

**Revendications**

1. Procédé de commande d'un système de purification de biotraitement continu comprenant une chromatographie continue (13 ; 20) configurée pour fonctionner avec au moins trois colonnes (A ; B ; C ; N) et configurée pour une purification continue dans une opération cyclique, dans lequel la purification continue est réalisée sur un échantillon comprenant un produit cible présentant des caractéristiques souhaitées, **caractérisé en ce que** le procédé comprend :

   a) la détection (82) d'au moins un paramètre indiquant des caractéristiques du produit cible,
   b) et la commande (84) du système de purification de biotraitement pour satisfaire les caractéristiques souhaitées sur la base de l'au moins un paramètre détecté, moyennant quoi les caractéristiques cibles s'inscrivent dans une plage prédéterminée,
   dans lequel la chromatographie continue est exécutée sous la forme d'une chromatographie à contre-courant périodique,
   dans lequel des impuretés dans le produit cible sont détectées à l'étape a) après que l'échantillon a été traité dans la chromatographie continue (13 ; 20), et l'étape b) comprend le retraitement du produit cible avec les impuretés détectées, et
   dans lequel le retraitement du produit cible comprend le traitement du produit cible avec les impuretés détectées dans une colonne différente (A ; B ; C ; N) dans la chromatographie continue (13 ; 20) par rapport à la colonne (A ; B ; C ; N) utilisée lors du traitement précédent.

2. Procédé selon la revendication 1, dans lequel le système de purification de biotraitement comprend une étape d'inactivation virale (14) après la chromatographie continue (13 ; 20) et le retraitement du produit cible avec des impuretés détectées comprend un traitement par une autre étape d'inactivation virale (14).

3. Procédé selon l'une quelconque des revendications 1 à 2, dans lequel l'étape b) commande un processus en amont (85) dans le système de purification de biotraitement.

4. Procédé selon la revendication 3, dans lequel l'étape b) comprend la commande de la concentration du produit cible dans l'échantillon qui est introduit dans la chromatographie continue (13 ; 20).

5. Procédé selon la revendication 3, dans lequel le système de purification de biotraitement comprend un processus de culture cellulaire (11) et l'étape b) comprend la commande (85a) du processus de culture cellulaire (11) pour ajuster la composition de l'échantillon qui est introduit dans la chromatographie continue (13 ; 20).

6. Procédé selon l'une quelconque des revendications 1 à 2, dans lequel l'étape b) commande un processus en aval (86) dans le système de purification de biotraitement.

7. Procédé selon la revendication 6, dans lequel la chromatographie continue (13, 20) comprend des étapes de chargement, d'élution et de nettoyage de chaque colonne (A ; B ; C ; N) et l'étape b) comprend l'ajustement (86a) de l'étape d'élution sur la base de la concentration de produit cible dans l'échantillon qui est introduit dans la chromatographie continue (13 ; 20).

8. Procédé selon la revendication 6, dans lequel le système de purification de biotraitement comprend une étape de polissage (15) et dans lequel le procédé comprend en outre :

- la définition (81) d'une empreinte de produit cible,
- à l'étape a), l'obtention régulière d'une empreinte après que l'échantillon a été traité dans la chromatographie continue (13 ; 20), et la comparaison de l'empreinte de produit cible à l'empreinte obtenue régulièrement de l'échantillon pour identifier des écarts, et
- à l'étape b), l'ajustement (86b) de l'étape de polissage (15) en réponse aux écarts identifiés.

9. Procédé selon la revendication 8, dans lequel l'empreinte de produit cible est définie par la composition du produit cible et/ou des impuretés détectées dans le produit cible.

10. Procédé selon la revendication 8 ou 9, dans lequel des empreintes sont obtenues par spectrométrie.

11. Procédé selon l'une quelconque des revendications 1 à 10, dans lequel le procédé comprend en outre la réalisation (83) d'une analyse de tendance en temps réel de chacun parmi l'au moins un paramètre détecté pour identifier un écart par rapport aux caractéristiques souhaitées du produit cible avant l'étape b), et la commande du système de purification de biotraitement sur la base de l'écart identifié.

12. Procédé selon la revendication 11, dans lequel la quantité purifiée du produit cible est mesurée à l'étape a), et l'analyse de tendance est réalisée dans le temps.

13. Système de purification de biotraitement comprenant une chromatographie continue (13 ; 20) configurée pour fonctionner en tant que chromatographie à contre-courant périodique avec au moins trois colonnes (A ; B ; C ; N) et configurée pour une purification continue dans une opération cyclique, dans lequel la purification continue est réalisé sur un échantillon comprenant un produit cible présentant des caractéristiques souhaitées, dans lequel le système de purification de biotraitement est en outre configuré pour :

- détecter (82) au moins un paramètre indiquant des caractéristiques du produit cible,
- commander (84) le système de purification de biotraitement pour satisfaire les caractéristiques souhaitées sur la base de l'au moins un paramètre détecté, moyennant quoi les caractéristiques cibles s'inscrivent dans une plage prédéterminée,
dans lequel la détection (82) comprend en outre la détection d'impuretés dans le produit cible après que l'échantillon a été traité dans la chromatographie continue (13 ; 20), et la commande (84) comprend en outre le retraitement du produit cible avec les impuretés détectées, et
dans lequel le retraitement du produit cible comprend le traitement du produit cible avec les impuretés détectées dans une colonne différente (A ; B ; C ; N) dans la chromatographie continue (13 ; 20) par rapport à la colonne (A ; B ; C ; N) utilisée lors du traitement précédent.

14. Système de purification de biotraitement selon la revendication 13, configuré en outre pour réaliser une analyse de tendance en temps réel de chacun des au moins un paramètre détecté pour identifier un écart par rapport aux caractéristiques souhaitées du produit cible, et la commande du système de purification de biotraitement est en outre basée sur les écarts identifiés.

15. Système de purification de biotraitement selon la revendication 13 ou 14, dans lequel un processus en aval, ou un processus en amont, est commandé dans le système de purification de biotraitement.

16. Programme informatique pour commander un système de purification de biotraitement, comprenant des instructions qui, lorsqu'elles sont exécutées sur au moins un processeur, amènent l'au moins un processeur à mettre en œuvre le procédé selon l'une quelconque des revendications 1 à 12.

17. Support de stockage lisible par ordinateur portant un programme d'ordinateur pour commander un système de purification de biotraitement selon la revendication 16.

Fig. 1

Fig. 2

Step 1

A    B    C

## Fig. 3a

Step 2

A    B    C

## Fig. 3b

Step 3

A    B    C

## Fig. 3c

# Fig. 4a

# Fig. 4b

# Fig. 4c

# Fig. 4d

Fig. 5

Fig. 6

73b Control downstream process

| 70 | | 71 | | 72 | |
|---|---|---|---|---|---|

70

Cell Culture

71

Separation

72

Batchify

API

73a Control upstream process

# Fig. 7a

Amount

130

133

134

132

target characteristics

131 Desired Range

# Fig. 13

Fig. 8

90

94

| 2nd | CIP | Elution | 1st |

A   B   C   D

95 Edit method

96

Column A

Column B

Column C

Column D

97

| A | Performance | |
| B | Performance | |
| C | Performance | |
| D | Performance | |

98

| Total yield | 98% | |
| Baseline 989mAu | | |
| ΔUV | 45% | |

99 Evaluate in
Real Time

Time

92a  92b  93  91

## Fig. 9

Column A        UV Flowthrough

mAU
900
800
700
600
500
400
300
200
100
0
325    330    335    340

## Fig. 10a

Column B        Cond Regeneration

mS/cm
20
15
10
5
0
325    330    335    340

## Fig. 10b

Column C        UV Elution

mAU
4000
3500
3000
2500
2000
1500
1000
500
0
325    330    335    340

## Fig. 10c

Column D        UV BT - Baseline

mAU
1100
1050
1000
950
900
850
800
325    330    335    340

## Fig. 10d

100

94

| 1st | 2nd | CIP | Elution |
|-----|-----|-----|---------|

A B C D

95 Edit method

96

Column A

Column B

Column C

Column D

97
| A | Performance | |
| B | Performance | |
| C | Performance | |
| D | Performance | |

98
| Total yield | 98% | |
| Baseline | 989mAu | |
| ΔUV | 45% | |

99 Evaluate in
Real Time

Time

101a    101b    91

Fig. 11

102

Chromatogram

103 Select columns | A | B | C | D | Ref |

97
| A | Performance | |
| B | Performance | |
| C | Performance | |
| D | Performance | |

98
| Total yield | 98% | |
| Baseline | 989mAu | |
| ΔUV | 45% | |

104 Return to
main screen

Time

91

Fig. 12

Fig. 7b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010151214 A